# EUROPEAN PATENT SPECIFICATION

(11) **EP 0 558 894 B1**
(45) Date of publication and mention of the grant of the patent: **01.05.1996**
(21) Application number: 93100551.6
(22) Date of filing: 15.01.1993
(51) Int. Cl.: C07C 225/20, C07C 221/00, A61K 31/16, C08G 73/10

(54) **Squaric acid derivatives**
Quadratsäurederivate
Dérivés de l'acide quadratique

(30) Priority: 03.03.1992 US 845254
(43) Date of publication of application: 08.09.1993
(73) Proprietor: KYOWA HAKKO KOGYO CO., LTD., Chiyoda-ku, Tokyo-to (JP)
(72) Inventor: Yumino, Yasuhisa, Ichihara-shi, Chiba-ken (JP); Muto, Hiroyuki, Yokkaichi-shi, Mie-ken (JP); Ito, Yukiyoshi, Yokkaichi-shi, Mie-ken (JP)
(74) Representative: VOSSIUS & PARTNER

(56) References cited:
- CHEMICAL ABSTRACT, vol. 87, no. 19, November 7, 1977, Columbus, Ohio, USA H. EHRHARDT et al. "Amides and thioamides of squaric acid: syntheses and reactions" page 547, abstract-no. 151 727q
- ANGEWANDTE CHEMIE, INTERNATIONAL EDITION IN ENGLISCH, vol. 5, 1966 G. MAAHS et al. "Syntheses and derivatives of squaric acid" pages 888-893
- CHEMICAL ABSTRACTS, vol. 81, no. 24, December 16, 1974, Columbus, Ohio, USA E. W. NEUSE et al. "Poly(squa- ryl amides)" page 2, abstract-no. 152 677m

## Description

The present invention relates to novel squaric acid derivatized diamines which are useful as ultraviolet absorbents and as raw materials for synthesizing heat-resistant polyimide resins.

Previously known squaric acid derivatives are 1,2-dianilino squaric acid derivatives where the 4-position of the phenyl group is hydrogen or nitro [J. Org. Chem., 39, 3881 (1974)] or where such position is hydrogen, hydroxyl, or carboxyl [Angew. Chem. Internat. Edit., 5, 888 (1966)].

However, there exists a need for squaric acid derivatized diamines which are particularly useful as ultraviolet absorbents and as raw materials for synthesizing heat-resistant polyimide resins.

The main object of the present invention is to provide novel squaric acid derivatized diamines which are useful as ultraviolet absorbents and as raw materials for synthesizing heat-resistant polyimides.

This object as well as other objects and advantages of the present invention will become apparent to those skilled in the art from the following description.

According to the present invention, there are provided novel squaric acid derivatized diamines (SQDA) having the following general structural Formula (I) or a salt thereof: 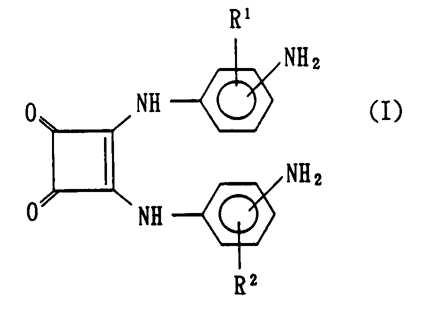
wherein R¹ and R² each is hydrogen or a lower alkyl group; and the two amino groups are independently attached to the meta-position or the para-position of the phenyl ring.

In the definitions of the groups in the formula (I), the lower alkyl group is a straight or branched alkyl group having 1 to 4 carbon atoms. Examples of the lower alkyl group include methyl, ethyl, propyl, isopropyl, butyl, isobutyl, sec-butyl, tert-butyl, and the like. Examples of the salts of the squaric acid derivatized diamines include salts of hydrohalogenic acids, such as hydrochloric acid and hydrobromic acid.

The general scheme for preparing the novel squaric acid derivatized diamines having the general structural formula (I) is shown in the following Schemes I and II. 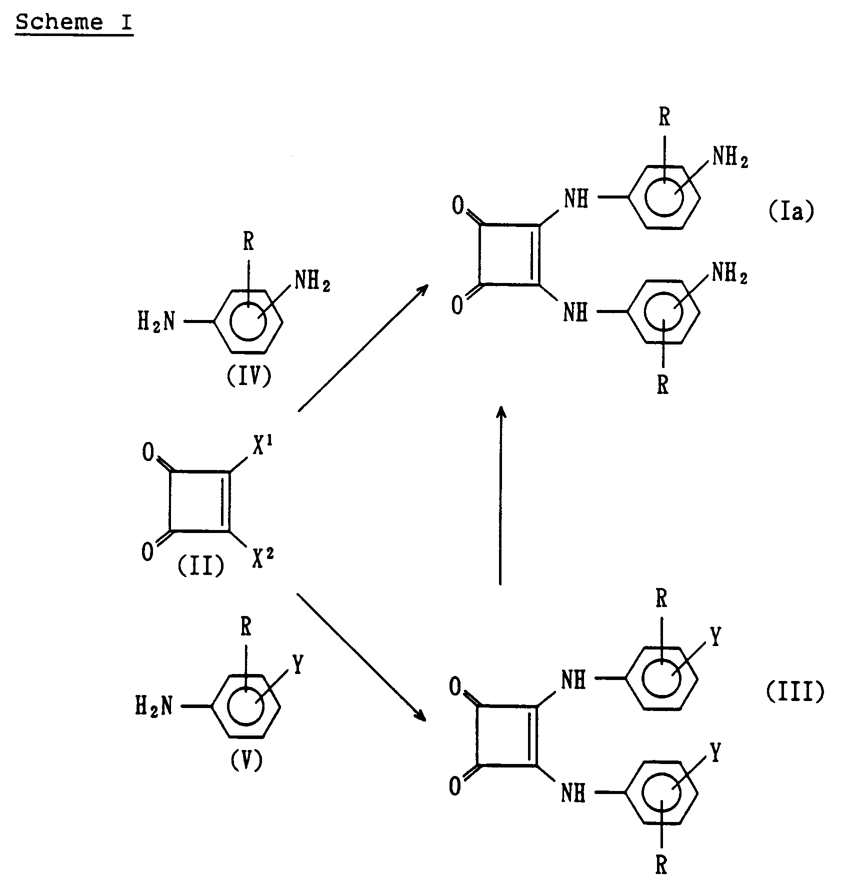
Wherein X¹ and X² each is a hydroxyl, a lower alkoxy, or a halogen group; R is the same as defined for R¹ and R²; and Y is a precursor for the amino group; and the amino group and Y are attached to the meta-position or the para-position of the phenyl ring.

The lower alkyl moiety in the lower alkoxy group for X¹ and X² is the same as the lower alkyl group defined for R¹ and R². Examples for Y, the precursor for the amino group, include alkanoylamino, such as acetylamino and propionylamino, benzyloxycarbonylamino, nitro, and the like.

The starting compound, Compound (II), can be synthesized according to known processes described in, for example, Tetrahedron Lett., 781 (1970); J. Am. Chem. Soc., 88, 1, 533 (1966); and Synthesis, 869 (1976).

The product, Compound (Ia), corresponds to the squaric acid derivatized diamines having the general Formula (I), where R¹ and R² are the same. In Scheme I, Compound (Ia) is obtained by reacting Compound (II) with a phenylenediamine derivative (IV) in an inert solvent. The amount of Compound (IV) employed is at least 2 molar equivalents, and preferably 2 to 10 molar equivalents, based on the amount of Compound (II). Examples of the inert solvent include alcohols, such as methanol and ethanol, ethers, such as diethyl ether, diisopropyl ether, tetrahydrofuran, and dioxane, aromatic hydrocarbons, such as benzene, toluene, and xylene, dimethylformamide, and the like, and mixtures thereof. The reaction between Compound (II) and Compound (IV) is carried out at a temperature of between 0°C and the boiling temperature of the solvent used. Normally, the reaction can be completed within a period of from 1 hour to 2 days.

As an alternative, Scheme I provides that instead of Compound (IV), Compound (V), which is a precursor to Compound (IV), can be used to react with Compound (II) to obtain Compound (III), a precursor to Compound (Ia). Using this reaction scheme has the advantage that the amount of by-products formed by the reaction is reduced. This reaction can be carried out under the same conditions as described for the reaction between Compound (II) and (IV). Compound (Ia) can then be obtained by subjecting the resulting Compound (III) to a hydrolysis or reduction reaction. 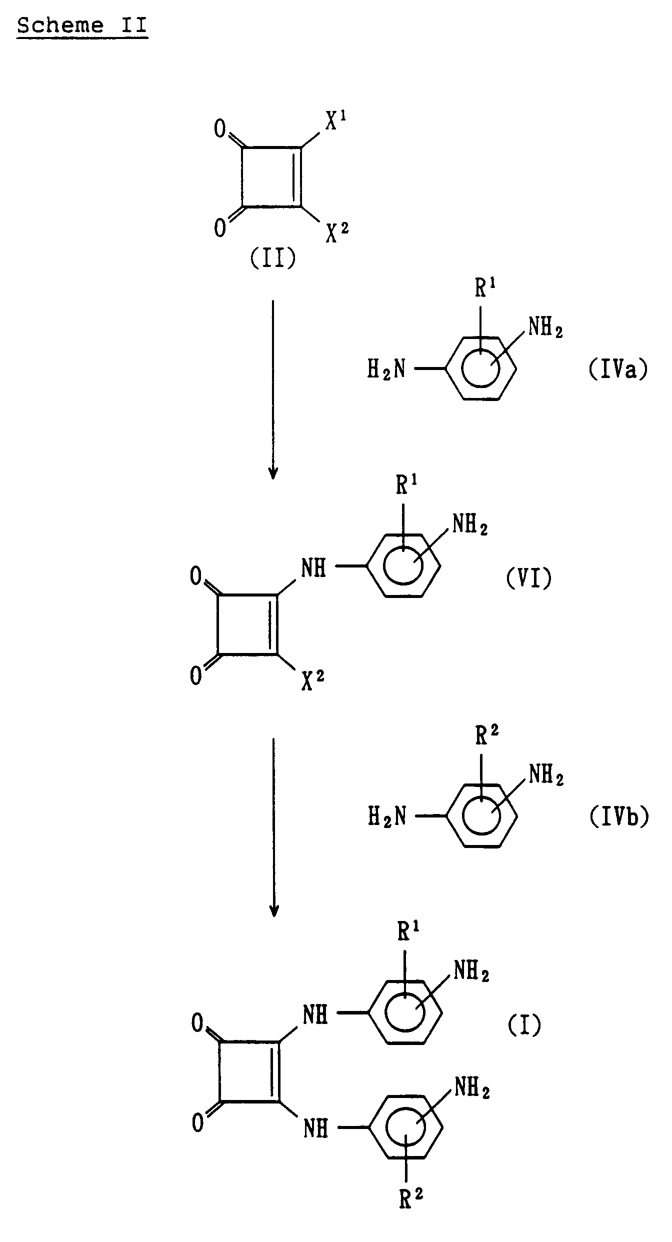
Wherein R¹, R², X¹, X² and the substitution position of the amino group are the same as defined previously.

This reaction scheme is particularly advantageous for synthesizing squaric acid derivatized diamines having the general Formula (I) where R¹ and R² are different from each other. The above reactions can be carried out under the same conditions as described in Scheme I. The amount of Compound (IVa) employed is preferably 1 to 2 molar equivalents based on the amount of Compound (II). The amount of Compound (IVb) employed is preferably 1 to 2 molar equivalents based on the amount of Compound (VI). As in Scheme I, instead of Compounds (IVa) and (IVb), Compounds (V) corresponding to the precursors for Compounds (IVa) and (IVb) can be used to react with Compound (II) to obtain a precursor to a compound having the Formula (I). The precursor can then be subjected to a hydrolysis or reduction reaction to obtain the product, a compound having the Formula (I).

In both Schemes I and II, the final product as well as the intermediate compounds formed can be isolated and purified by any of the conventional methods employed in organic synthetic chemistry, e.g., filtration, extraction, washing, drying, concentration, recrystallization, and chromatography. If desired, the intermediate compounds formed can be used in subsequent steps without purification.

If the product is synthesized in the unsalted form, to obtain a salt of the compound having the Formula (I), it can be converted into a salt by, for example, dissolving or suspending it in an appropriate organic solvent and adding an appropriate acid. When the product is synthesized in the salted form, the salted compound can be subjected to known purification or isolation processes to obtain the salted product.

Compounds having the Formula (I) and salts thereof can also be present in the form of addition products to water or various solvents, for example, those described in Scheme I. Such addition products are included within the scope of the present invention.

The compounds having the Formula (I) exhibit strong UV absorption peaks in the range of 290nm to 400nm. The compounds can be added to cosmetics or the like as an UV absorbent for protecting the skin from ultraviolet rays. They can also be added to plastics, such as polyolefins, as an agent for preventing the plastics from deteriorating. The compounds having the Formula (I) can also be used as raw materials for synthesizing heat-resistant polyimides.

The heat-resistant polyimides can be prepared by reacting SQDA with various aromatic dianhydrides. Examples of the dianhydrides that can be employed include 3,3',4,4'-benzophenone tetracarboxylic dianhydride (BTDA); pyromellitic dianhydride (PMDA); 4,4'-oxydiphthalic dianhydride (ODPA); and 3,3',4,4'-biphenyl tetracarboxylic dianhydride (BPDA). Other aromatic dianhydrides known to those skilled in the art may also be used.

The polymerizations of the various dianhydrides with SQDA can be carried out in N,N-dimethylacetamide (DMAc) at 7.45 to 15.00 percent solids (weight/weight) at ambient conditions. In addition to DMAc, other solvents known to those skilled in the art may also be employed.

The following examples and reference example are illustrative of the invention.

### EXAMPLE 1 Synthesis of 1,2-Bis(4-aminoanilino)cyclobutene-3,4-dione (Compound 1):

Compound 1 was prepared in the following manner. First, 6.50 g (60mmol) of p-phenylenediamine was dissolved in 50 ml of methanol. A solution of 1.70 g (10mmol) of diethyl squarate in 20 ml of methanol was added in a dropwise manner to the p-phenylenediamine solution at room temperature over a period of 1 hour. This addition was run under a nitrogen atmosphere. The resulting mixture was stirred for 3 hours at room temperature and allowed to stand overnight. A crystalline product formed in the reaction flask during this period and it was collected by filtration, washed with methanol and vacuum dried for 1 hour at 150°C to yield 2.50 g of Compound 1 as a greenish-orange solid.

| Elemental analysis: | | | | |
|---|---|---|---|---|
| Calculated for C₁₆H₁₄N₄O₂: | C, 65.30; | H, 4.79; | N, 19.04; | O, 10.87. |
| Found: | C, 64.81; | H, 4.70; | N, 18.45; | O, 11.25. |

Infrared Spectrum (KBr), cm⁻¹: 1795, 2700-3700
Proton NMR (DMSO-d₆)δ(ppm): 4.97 (s,4H); 6.60 (d,J=8.8Hz,4H); 7.18 (d,J=8.8Hz,4H); 9.42 (s,2H).
Carbon-13 NMR (DMSO-d₆)δ(ppm): 114.4 x 2; 120.1 x 2; 128.0; 145.1; 164.5; 180.8.
UV:λₘₐₓ (εₘₐₓ):
In acetonitrile : water = 1 : 1 :
279.0 nm (25,300), 345.2 nm (28,600).
In dimethylformamide:
328.0 nm (26,600), 362.0 nm (27,700).

### Example 2 Synthesis of 1,2-Bis(3-aminoanilino)cyclobutene-3,4-dione (Compound 2):

Compound 2 was prepared in the following manner. First, 32.4 g (0.3 mol) of m-phenylenediamine was dissolved in 300 ml of methanol. A solution of 8.6 g (0.05 mol) of diethyl squarate in 50 ml of methanol was added in a dropwise manner to the m-phenylenediamine solution at room temperature over a period of 30 minutes. This addition was run under a nitrogen atmosphere. The resulting mixture was stirred for 3 hours at room temperature and allowed to stand overnight. A crystalline product formed in the reaction flask during this period and it was collected by filtration, washed with methanol, purified by column chromatography and vacuum dried for one hour at 150°C to yield 1.0 g of Compound 2 as a greenish-orange solid.

| Elemental analysis: | | | | |
|---|---|---|---|---|
| Calculated for C₁₆H₁₄N₄O₂: | C, 65.30; | H, 4.79; | N, 19.04; | O, 10.87. |
| Found: | C, 64.90; | H, 4.71; | N, 18.78; | O, 11.61. |

Infrared Spectrum (KBr), cm⁻¹: 1790, 2700-3700
Proton NMR (DMSO-d₆)δ(ppm): 5.21 (s,4H); 6.32 (m,2H); 6.61 (m,2H); 6.76 (m,2H); 7.01 (m,2H); 9.59 (s,2H).
Carbon-13 NMR (DMSO-d₆)δ(ppm): 103.6; 106.1; 109.5; 129.7; 139.2; 149.6; 165.5; 181.2.
UV: λₘₐₓ (εₘₐₓ): 346.0nm (34,000) in dimethylformamide.

### Reference Example 1

### 1. Reaction of 3,3',4,4'-benzophenone tetracarboxylic dianhydride (BTDA) with Compound 1

In a serum bottle, 0.58858 g of Compound 1 prepared in Example 1 was admixed with 0.6445 g of BTDA (0.002 molar reaction). Seven grams of DMAc were added thereto and the solution was stirred at ambient temperature. After approximately 5 minutes a build up in viscosity was observed. The solution was stirred for an additional 1 hour. Inherent viscosity was found to be 1.19 dl/g when run at 25°C at 0.5 % concentration in DMAc.

The solution was poured onto a soda-lime glass plate and doctored to a thickness of approximately 0.015 inches; placed in a nitrogen purged dry box and the solvent was evaporated. After several hours the coated glass plate was placed in a forced air oven and was heated to 200°C to ensure complete cure of the polymeric coating. The resulting reddish-brown coating adhered tenaciously to the glass.

The coated glass was immersed in warm water for 1 hour and a sharp razor blade was used to initiate removal of the polymer from the glass. As the result, the polymer was a free standing film that was very flexible and tough. This film was heated for an additional hour at 300°C and still remain flexible and tough.

### 2. Thermal stabilities and physical characteristics of polyimide.

The thermal stabilities of the polyimide formed by reaction between BTDA and Compound 1 were examined by heating the polymer in air from ambient temperature to 600°C at a raising rate of 2.5 °C/minute. The resultant thermal stabilities based on the temperatures where 5, 25, and 50 percent weight losses were found were very high. Table 1 below summarizes the cure schedule, weight loss profile, and physical characteristics of the polyimide that was synthesized.

**Table I**

| Thermal Stability of Polyimide Films Weight Loss Temperature °C | | | | |
|---|---|---|---|---|
| Cure | 5% | 25% | 50% | Physical Character |
| 200°C | 283 | 490 | 531 | Orange/brown; Flexible |
| 400°C | 337 | 491 | 526 | Red/brown; Flexible |

All films have a translucent appearance after 200°C cure which indicates crystallinity; however, no melting is noted on differential scanning calorimetry (DSC). Translucency persists after 300°C cure.

Because of its excellent adhesion and flexibility, the polyimide formed is useful as a high temperature adhesive.

The foregoing specific examples are exemplary and are not to be considered as exhaustive, but merely to illustrate the invention without serving as limitations thereon.

## Claims

1. A squaric acid derivatized diamine having the following general structural formula or a salt thereof: 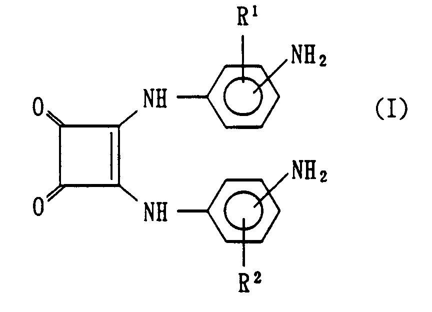 wherein R¹ and R² each is hydrogen or a lower alkyl group wherein the lower alkyl group is a straight or branched alkyl group having 1 to 4 carbon atoms and the two amino groups are independently attached to the meta-position or the para-position of the phenyl ring.

2. The squaric acid derivatized diamine according to Claim 1, wherein the salt is selected from salts of hydrohalogenic acids.

3. The squaric acid derivatized diamine according to Claim 2, wherein the hydrohalogenic acids are selected from hydrochloric acid and hydrobromic acid.

4. A cosmetic composition comprising a squaric acid derivatized diamine according to anyone of Claims 1 to 3 and a cosmetically acceptable excipient.

5. A plastic composition comprising a squaric acid derivatized diamine according to anyone of Claims 1 to 3 and a polyolefin.

6. Use of squaric acid derivatized diamines according to anyone of Claims 1 to 3 for the preparation of heat-resistant polyimides by reacting them with aromatic dianhydrides.

7. A process for producing a squaric acid derivatized diamine according to anyone of Claims 1 to 3 by
a) reacting a compound of the general formula (II) 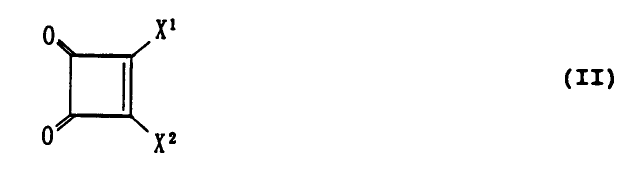 wherein X¹ and X² each is a hydroxyl, a lower alkoxy or a halogen group
with a compound of the general formula (IVa) 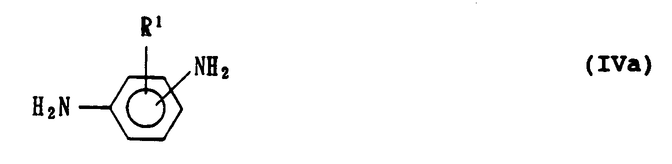 and then
with a compound of the general formula (IVb) 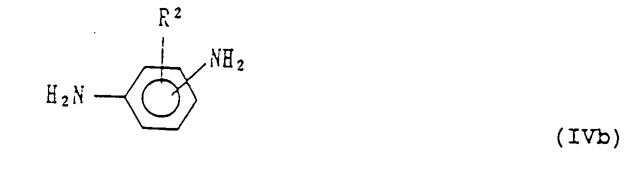 wherein R¹ and R² have the same meanings as in Claim 1 and the two amino groups are independently attached to the meta-position or the para-position of the phenyl ring, or
b) reacting a compound of the general formula (II) with a compound of the general formula (V) 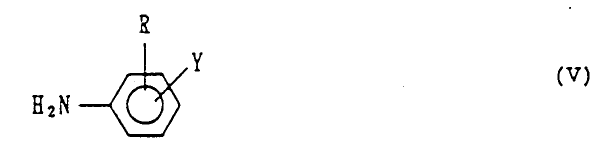 wherein Y is a precursor for an amino group; and the amino group and Y are attached to the meta-position or the para-position of the phenyl ring and R is the same as defined for R¹ and R² followed by converting the obtained compound of the general formula (III) 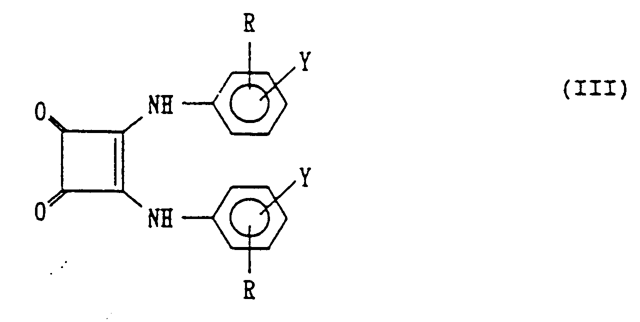 to a compound (I)
and by optionally forming the corresponding salt.

## Patentansprüche

1. Quadratsäurediaminderivat der nachstehenden allgemeinen Strukturformel: 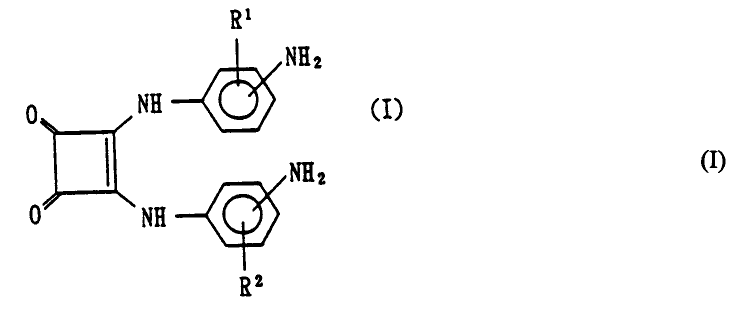 in der R¹ und R² jeweils ein Wasserstoffatom oder ein Niederalkylrest sind, wobei der Niederalkylrest ein unverzweigter oder verzweigter Alkylrest aus 1 bis 4 Kohlenstoffatomen ist, und die zwei Aminogruppen unabhängig voneinander in m-Stellung oder p-Stellung des Phenylringes gebunden sind, oder ein Salz davon.

2. Quadratsäurediaminderivat gemäß Anspruch 1, wobei das Salz aus Salzen von Halogenwasserstoffsäuren ausgewählt ist.

3. Quadratsäurediaminderivat gemäß Anspruch 2, wobei die Halogenwasserstoffsäuren aus Chlorwasserstoffsäure und Bromwasserstoffsäure ausgewählt sind.

4. Kosmetikzusammensetzung, umfassend ein Quadratsäurediaminderivat gemäß einem der Ansprüche 1 bis 3 und ein kosmetisch verträgliches Excipiens.

5. Kunststoffzusammensetzung, umfassend ein Quadratsäurediaminderivat gemäß einem der Ansprüche 1 bis 3 und ein Polyolefin.

6. Verwendung eines Quadratsäurediaminderivates gemäß einem der Ansprüche 1 bis 3 zur Herstellung hitzebeständiger Polyimide durch ihre Umsetzung mit aromatischen Dianhydriden.

7. Verfahren zur Herstellung eines Quadratsäurediaminderivates gemäß einem der Ansprüche 1 bis 3 durch
a) die Umsetzung einer Verbindung der allgemeinen Formel (II) 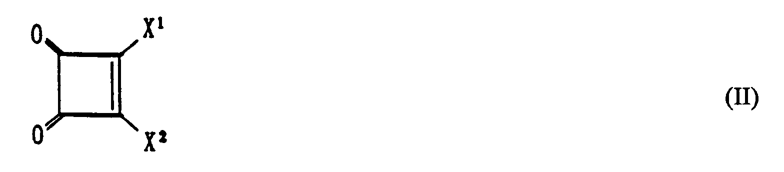 in der X¹ und X² jeweils eine Hydroxylgruppe, ein Niederalkoxyrest oder ein Halogenatom sind, mit einer Verbindung der allgemeinen Formel (IVa) 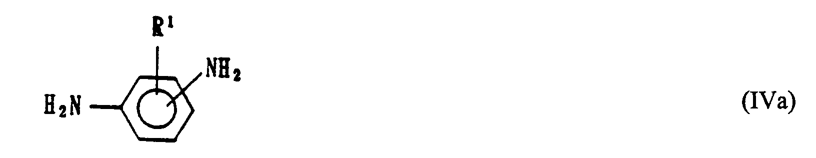 und anschließend mit einer Verbindung der allgemeinen Formel (IVb) 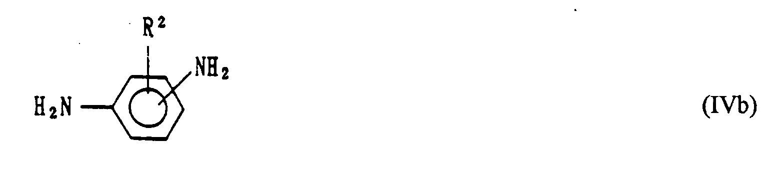 in der R¹ und R² dieselben Bedeutungen wie in Anspruch 1 haben, und die zwei Aminogruppen unabhängig voneinander in m-Stellung oder p-Stellung des Phenylringes gebunden sind, oder
b) die Umsetzung einer Verbindung der allgemeinen Formel (II) mit einer Verbindung der allgemeinen Formel (V) 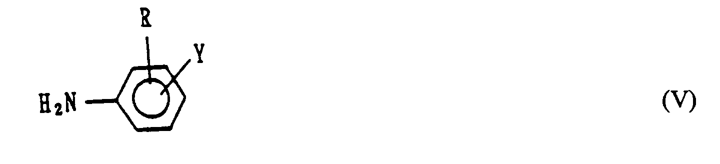 in der Y eine Vorstufe einer Aminogruppe ist, und die Aminogruppe und Y in m-Stellung oder p-Stellung des Phenylringes gebunden sind, und R wie für R¹ und R² definiert ist, und anschließend die Umwandlung der erhaltenen Verbindung der allgemeinen Formel (III) 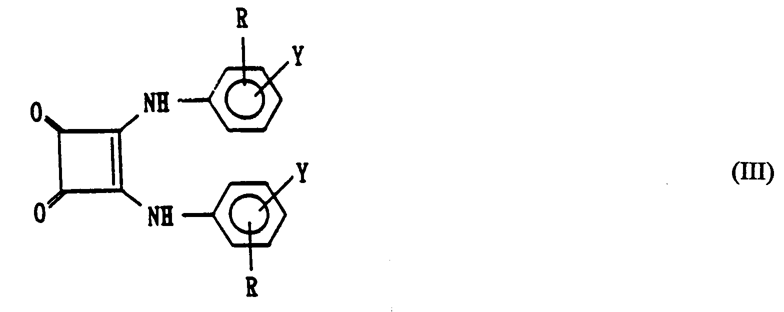 in eine Verbindung (I) und gegebenenfalls durch die Bildung des entsprechenden Salzes.

## Revendications

1. Diamine dérivée de l'acide quadratique, ayant la formule structurale générale suivante, ou un sel de celle-ci : 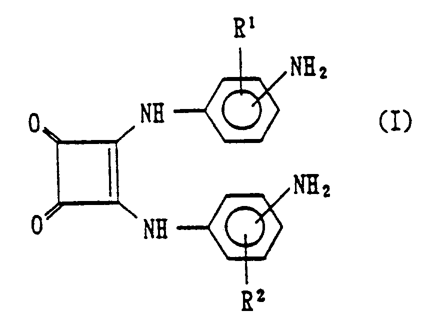 dans laquelle R¹ et R² représentent chacun hydrogène ou un groupe alkyle inférieur, le groupe alkyle inférieur étant un groupe alkyle linéaire ou ramifié ayant 1 à 4 atomes de carbone, et les deux groupes amino sont indépendamment fixés en position méta ou en position para du noyau phényle.

2. Diamine dérivée de l'acide quadratique, selon la revendication 1, dans laquelle le sel est choisi parmi les sels d'acides halohydriques.

3. Diamine dérivée de l'acide quadratique, selon la revendication 2, dans laquelle les acides halohydriques sont choisis parmi l'acide chlorhydrique et l'acide bromhydrique.

4. Composition cosmétique comprenant une diamine dérivée de l'acide quadratique, telle que définie à l'une quelconque des revendications 1 à 3, et un excipient cosmétiquement acceptable.

5. Composition plastique comprenant une diamine dérivée de l'acide quadratique, telle que définie à l'une quelconque des revendications 1 à 3, et une polyoléfine.

6. Utilisation de diamines dérivées de l'acide quadratique, telles que définies à l'une quelconque des revendications 1 à 3, pour la préparation de polyimides résistants à la chaleur par réaction de celles-ci avec des dianhydrides aromatiques.

7. Procédé de fabrication d'une diamine dérivée de l'acide quadratique, telle que définie à l'une quelconque des revendications 1 à 3, par :
(a) réaction d'un composé de la formule générale (II) : 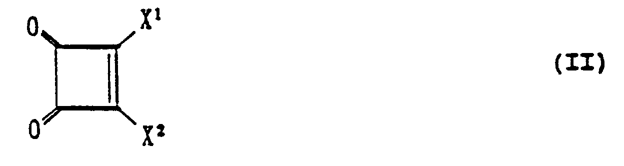 dans laquelle X¹ et X² représentent chacun un groupe hydroxyle, un groupe alcoxy inférieur ou un groupe halogène,
avec un composé de la formule générale (IVa) : 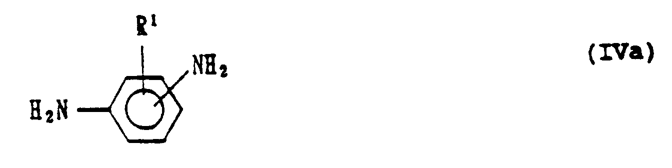 puis avec un composé de la formule générale (IVb) : 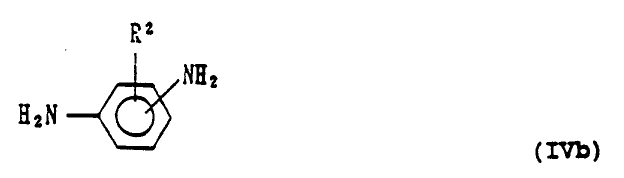 dans laquelle R¹ et R² ont la même signification que dans la revendication 1 et les deux groupes amino sont indépendamment fixés en position méta ou en position para du noyau phényle, ou
(b) réaction d'un composé de la formule générale (II) avec un composé de la formule générale (V) : 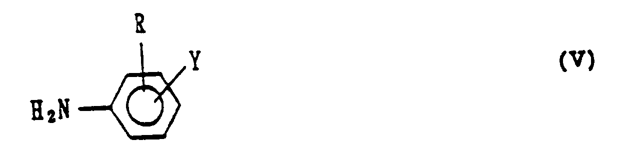 dans laquelle Y est un précurseur pour un groupe amino; et le groupe amino et Y sont fixés en position méta ou en position para du noyau phényle, et R est le même que celui défini pour R¹ et R²,
en faisant suivre par la conversion du composé obtenu de la formule générale (III) : 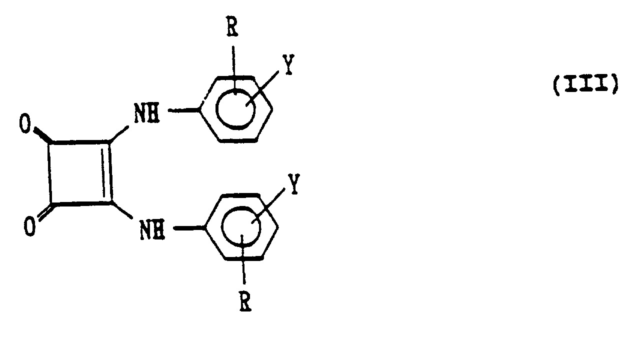 en un composé (I)
et par la formation facultative du sel correspondant.
